# EUROPEAN PATENT SPECIFICATION

(11) **EP 2 881 404 B1**
(45) Date of publication and mention of the grant of the patent: **28.02.2018**
(21) Application number: 13825506.2
(22) Date of filing: 02.08.2013
(51) Int. Cl.: C08B 37/10, A61K 31/702

(54) **METHOD FOR OBTAINING LOW MOLECULAR WEIGHT AND VERY LOW MOLECULAR WEIGHT HEPARINS**
VERFAHREN ZUR GEWINNUNG VON HEPARINEN MIT NIEDRIGEM MOLEKULARGEWICHT UND SEHR NIEDRIGEM MOLEKULARGEWICHT
PROCÉDÉ D'OBTENTION D'HÉPARINES DE BAS ET TRÈS BAS POIDS MOLÉCULAIRE

(30) Priority: 02.08.2012 ES 201231257
(43) Date of publication of application: 10.06.2015
(73) Proprietor: Laboratorios Farmacéuticos Rovi, S.A., 28037 Madrid (ES)
(72) Inventor: FRANCO RODRIGUEZ, Guillermo, E-28037 Madrid (ES); GUTIERRO ADURIZ, Ibon, E-28037 Madrid (ES)
(74) Representative: Carpintero Lopez, Francisco
(86) International application number: PCT/ES2013/070575
(87) International publication number: WO 2014/020227

(56) References cited:
- EP-A1- 1 070 503
- EP-A1- 2 548 561
- ES-A6- 2 003 197
- ES-T3- 2 347 134
- ES-T3- 2 383 597
- US-A- 5 707 973
- Y. XU ET AL: "Chemoenzymatic Synthesis of Homogeneous Ultralow Molecular Weight Heparins", SCIENCE, vol. 334, no. 6055, 27 October 2011 (2011-10-27), pages 498-501, XP055126707, ISSN: 0036-8075, DOI: 10.1126/science.1207478
- VISKOV C ET AL: "Description of the chemical and pharmacological characteristics of a new hemisynthetic ultra-low-molecular-weight heparin, AVE5026", JOURNAL OF THROMBOSIS AND HAEMOSTASIS, WILEY INTERSCIENCE, ENGLAND, vol. 7, no. 7, 1 July 2009 (2009-07-01), pages 1143-1151, XP002545508, ISSN: 1538-7836, DOI: 10.1111/J.1538-7836.2009.03447.X
- VISKOV C ET AL.: 'Description of the chemical and pharmacological characteristics of a new hemisynthetic ultra-low-molecular-weight heparin, AVE5026' JOURNAL OF THROMBOSIS AND HAEMOSTASIS vol. 7, no. 7, 01 July 2009, pages 1143 - 1151, XP002545508

## Description

### OBJECT OF THE INVENTION

This invention refers to the process for preparing low molecular weight heparins (LMWH) and very low molecular weight heparins (VLMWH).

### BACKGROUND OF THE INVENTION

Heparin is a polysaccharide in the glycosaminoglycan family, formed by alternatively linked uronic acid (L-iduronic or D-glucuronic acid) and D-glucosamine units. L-iduronic acid can be 2-O-sulfated and D-glucosamine can be N-sulfated and/or 6-O-sulfated and to a lesser extent N-acetylated or 3-O-sulfated (Mapping and quantification of the major oligosaccharides component of heparin. Linhardt RJ, Rice KG, Kim YS et al. Biochem J 1988; 254 :781-787). Heparin is preferably used as the sodium salt, but can also be used as a salt of other alkali or alkaline earth metals, and is mainly used as an antithrombotic and anticoagulant medicine (Anticoagulant therapy for major arterial and venous thromboembolism. Tran HAM, Ginsberg JS. Basic principles and clinical practice. Colman RW, Marder VJ, Clowes AW, George JN, Goldhaber SZ (Ed). Lippincott Williams and Wilkins; 2006:1673-1688).

Heparins can be classified according to their molecular weight into unfractionated heparins (UFH), low molecular weight heparins (LMWH) and very low molecular weight heparins (VLMWH). The LMWH and VLMWH come from depolymerisation of the original UFH.

The antithrombotic action of the LMWHs, as with other heparins, requires the presence of antithrombin III (ATIII). The heparin binds to antithrombin via a specific pentasaccharide sequence forming a ternary complex, LMWH-ATIII-Factor Xa. This inhibition is known as antithrombotic action. By contrast, in anticoagulant action, thrombin neutralisation requires the formation of a ternary complex, LMWH-ATIII-Factor IIa, for which at least 18 saccharides are required.

The improvement of LMWH with respect to sodium heparin is due to a large extent on the enrichment in the ATIII activating site of the former compared to the latter.

In unfractionated heparin, as well as various LMWH or VLMWH obtained by known methods of depolymerisation (enzymatic, nitrous acid, β-elimination), there is a measure that enables determination of the amount of pentasaccharide in the general structure through quantification of the content of a specific and exclusive disaccharide unit of the pentasaccharide corresponding to D-glucuronic acid (G) linked to N-sulfo-3-O-sulfo-D-glucosamine (ANS, 3S). This measure enables obtaining a good correlation between the percentage of this disaccharide present and the content of pentasaccharide in the heparin structure and therefore of the anti-FXa activity of the heparin in question (Low molecular weight heparins: structural differentiation by two-dimensional nuclear magnetic resonance spectroscopy. Guerrini M, Guglieri S, Naggi A, Sasisekharam R and Torri G. Seminars in Thrombosis and Hemostasis 2007; 33: 478-487).

In the state of the art, various methods have been described for the preparation of LMWH. The document of patent EP01955436 describes a process for obtaining LMWH by a process comprising the stages of conversion of a heparin salt into a benzethonium heparinate salt, esterification of the benzethonium heparinate with benzyl chloride, purification and obtaining the sodium salt of the heparin benzyl ester, conversion of the heparin benzyl ester into the benzethonium salt, depolymerization with a strong base in organic medium, saponification of the ester and, if necessary, purification of the product. In that invention it was necessary to first form a benzyl ester of heparin intermediate that was used in turn to generate the corresponding quaternary ammonium heparinate, which was the substrate on which the base acted. In the present invention, the process described is simpler, avoiding the need to prepare the benzyl ester intermediate with the logical saving both in cost and time for the process.

The document of patent EP1070503 describes a process for obtaining low molecular weight heparins with the following properties: average molecular weight 2000-4000 Daltons, anti-FXa activity between 100 and 150 IU/mg, anti-FIIa activity less than or equal to 10 IU/mg. These LMWH are obtained by treatment of benzalkonium heparinate with a base in non-aqueous medium consisting of the following stages: transalification of sodium heparin into benzalkonium heparinate, first depolymerisation with base in organic medium, conversion of the benzalkonium salt into a sodium salt, formation of the benzalkonium salt of the intermediate obtained after the first depolymerisation, second depolymerisation with base in organic medium, transalification of the benzalkonium salt into the sodium salt and final purification.

Finally, the document of patent ES2003197 describes the same process as in the patent document EP1070503, previously mentioned, except that the salt that is formed is a benzethonium heparinate. The base used in this process is Triton B, quaternary ammonium hydroxide, and the document describes that any quaternary ammonium hydroxide can be used, without giving any indication how the base may affect the process and explaining that it is not a crucial element and any can be used.

Therefore, from the state of the art, it is concluded that it would be very useful for the industry to develop an alternative efficient process for the preparation of LMWH and VLMWH that would provide a process that preserves to the maximum the specific ATIII pentasaccharide sequence and that eliminates stages such as esterification of benzethonium heparinate with benzyl chloride, transalification of the salt of the heparin benzyl ester into a benzethonium salt and subsequent saponification of this ester.

### DESCRIPTION OF THE INVENTION

The inventors have found a new process for obtaining LMWH and VLMWH with high yield and high region selectivity, which makes this process more selective. The process of the invention is also simpler than the other known processes.

The inventors have shown that in the processes of hemisynthesis of LMWH and VLMWH by β-elimination mechanisms, the specific use of benzalkonium heparinate as a substrate for depolymerisation endows on the action of strong and sterically hindered bases the ability to preserve the pentasaccharide activator of ATIII. This pentasaccharide contains a trisulfated monosaccharide in its structure and is therefore more sterically hindered than other possible cleavage positions of the molecule with monosaccharides of a lower degree of sulfation.

The process is characterised by a first transalification stage, that is a conversion reaction of one salt into another salt, depolymerisation with a phosphazene base or a base derived from guanidine and a final transalification.

Therefore, a first aspect of the invention refers to a process for obtaining the compositions of the invention that comprise the stages of:
a) transalification of heparin of an alkali or alkaline earth metal into benzalkonium heparinate;
b) depolymerisation in organic medium with the addition of a phosphazene base or a base derived from guanidine; and
c) transalification to form a salt of an alkali or alkaline earth metal.

### DESCRIPTION OF THE FIGURES

This description is complemented with a set of figures, illustrating a preferred embodiment, that are not limiting of the invention in any way.

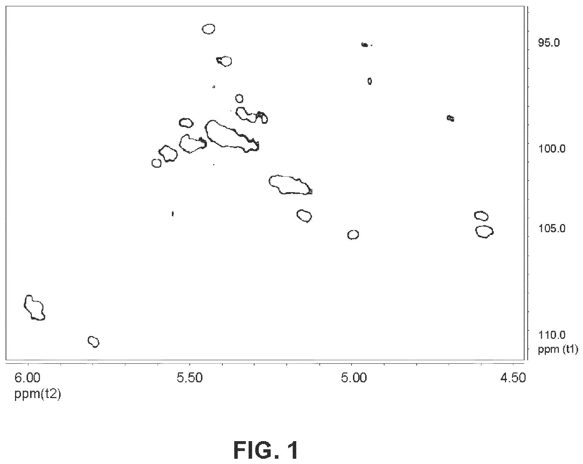

Figure 1: Representation of the region of anomeric signals (H1-C1 correlations) of the 13C-1H HSQC spectrum of the product described in example 4, depolymerised with Triton B, recorded at 298 K in deuterated water (D2O). The spectrum shows five correlation peaks corresponding to the five monosaccharides of the pentasaccharide. ANSred, N-sulfo-D-glucosamine of the reducing end; I2S, L-iduronic acid 2-sulfate; ANS,3S, N-sulfo-3-O-sulfo-D-glucosamine; G-(ANS,3S), D-glucuronic acid that precedes the ANS,3S unit and ANS-(G), N-sulfo-D-glucosamine that precedes the D-glucuronic acid ring. The H1-C1 correlation peak of the G-(ANS,3S) unit has been highlighted with a circle because it is the most characteristic signal of the pentasaccharide and will be taken as a reference for quantifying the presence of the pentasaccharide in various samples.

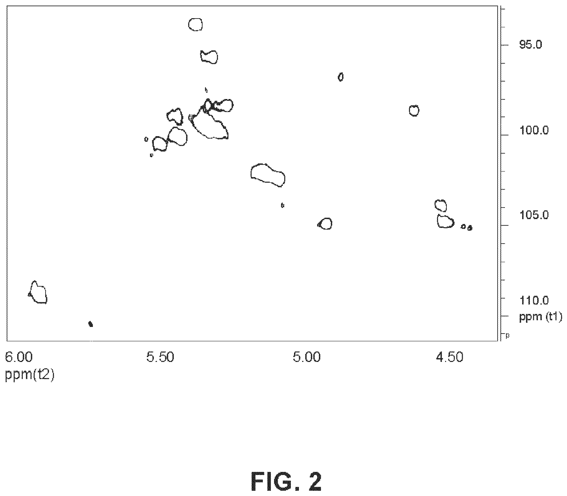

Figure 2: Representation of the region of the anomeric signals (H1-C1 correlations) of the 13C-1H HSQC spectrum of the product described in example 4 depolymerised with BEMP, recorded at 298 K in deuterated water (D2O). The spectrum shows five correlation peaks corresponding to the five monosaccharides of the pentasaccharide. ANSred, N-sulfo-D-glucosamine of the reducing end; I2S, L-iduronic acid 2-sulfate; ANS,3S, N-sulfo-3-O-sulfo-D-glucosamine; G-(ANS,3S), D-glucuronic acid that precedes the ANS,3S unit and ANS-(G), N-sulfo-D-glucosamine that precedes the D-glucuronic acid ring. The H1-C1 correlation peak of the G-(ANS,3S) unit has been highlighted with a circle because it is the most characteristic signal of the pentasaccharide and will be taken as a reference for quantifying the presence of the pentasaccharide in various samples.

### DETAILED DESCRIPTION OF THE INVENTION

In this invention, the term "phosphazene base or a base derived from guanidine" refers to strong neutral and slightly nucleophilic bases such as P1-t-Bu, P2-t-Bu, t-BuP4 and BEMP (2-tert-butylimino-2-diethylamino-1,3-dimethyl-perhydro-1,3-diaza-2-phosphorine) and TTMG (2-tert-butyl-1,1,3,3-tetramethylguanidine) strong bases in aprotic solvents (MeCNpKa BEMP= 27.6, DMSOpKa t-BuP4H+= 32).
As mentioned above, a first aspect of the invention refers to a process for obtaining VLMWH and/or LMWH comprising the steps:
a) transalification of heparin of an alkali or alkaline earth metal into benzalkonium heparinate;
b) depolymerisation in organic medium with the addition of a phosphazene base or a base derived from guanidine; and
c) transalification to form a salt of an alkali or alkaline earth metal.

If the final product is to be purified between step b) and step c), an organic and/or inorganic peroxide is added.

Preferably the heparin of an alkali or alkaline earth metal of step a) is an unfractionated heparin of an alkali or alkaline earth metal.

Preferably, the peroxide used is hydrogen peroxide. Other examples of organic peroxides are: methyl ethyl ketone peroxide, benzoyl peroxide, acetone peroxide. Examples of inorganic peroxides are compounds characterised by the presence of (O₂)⁻² with metals of groups IA and IIA.

Preferably, the product obtained after step c) is purified. The purification of the product is preferably performed by dissolving the product in water at a concentration of between 5% and 20% (w/v), adjusting the pH to a value between 6 and 7.5, addition of sodium chloride at a concentration of between 3% and 15% (w/v) and precipitation with alcohol. Preferably the product is dissolved in water at a concentration of between 7.5% and 12.5% (w/v), adjusting the pH to a value of between 6.5 and 7.25, addition of sodium chloride to a concentration of between 5% and 10% (w/v) and precipitation with methanol.

The depolymerisation step is performed in organic solvent, preferably the organic solvent is dichloromethane, dichloroethane, chloroform, dimethylformamide and/or formamide. Particularly the organic solvent is dichloromethane at a temperature of between 20 and 40 °C.

Step b) of the depolymerisation is preferably performed in at least two successive additions of the base. Particularly, it is performed in three successive additions of the base.

The weight:volume ratio between benzalkonium heparinate and the base for each addition is between 1:0.5 and 1:0.05 and preferably between 1:0.3 and 1:0.1.

The weight:volume ratio between benzalkonium heparinate and hydrogen peroxide is between 1:1 and 1:0.01, and preferably between 1:0.2 and 1:0.05.

Preferably, the transalification of step c) is performed by precipitation from the reaction medium with an alcoholic sodium acetate solution, preferably with a 10% (w/v) solution of sodium acetate in methanol.

A second aspect of this invention is the product obtained through the process of the invention that contains a higher pentasaccharide fraction and therefore higher antithrombotic activity.

### EXAMPLES

The following specific examples are provided below to serve to illustrate the nature of this invention. These examples are included for illustration purposes only and should not be interpreted as limitations of the invention claimed herein.

### EXAMPLE 1

100 g of sodium heparin were dissolved in 725 ml of water and 880 ml of a solution of benzalkonium chloride in water (50% w/v) were added. The product was filtered, washed with water and lyophilised. 233 g of benzalkonium heparinate were obtained.

10 g of benzalkonium heparinate obtained in the previous step were dissolved in 30 ml of dichloromethane and warmed to 35 °C. To this solution was added 2-tert-butylimino-2-diethylamino-1,3-dimethyl-perhydro-1,3-diaza-2-phosphorine, BEMP, in three portions.
- 1.72 ml of BEMP. The reaction was maintained at 35 °C for 8 hours.
- 1.72 ml of BEMP. The reaction was maintained at 35 °C for 16 hours.
- 1.72 ml of BEMP. The reaction was maintained at 35 °C for 8 hours.

1 ml of hydrogen peroxide were added to the solution and the reaction maintained at 35 °C for 16 hours and then precipitated by adding 60 ml of sodium acetate in methanol (10% w/v). The precipitate was collected by centrifugation and washed with methanol. The product obtained was dissolved in 5 ml of water, neutralised, sodium chloride added to a concentration of 10% w/v and precipitated with 125 ml of methanol. The precipitate was collected by filtration, washed in methanol and dried in a vacuum oven at 35 °C. The product weighing 3.39 g was obtained.

2 g of the depolymerised product was dissolved in 14.5 ml of water and 17.6 ml of benzalkonium chloride at 50% w/v in water were added. The product was filtered, washed with water and lyophilised. The 4.84 g of product obtained were dissolved in 14.5 ml of dichloromethane and warmed to 35 °C. 0.83 ml of BEMP were added and the reaction maintained at 35 °C for 16 hours. After this time, the product was precipitated with 26 ml of sodium acetate solution in methanol (10% w/v). The precipitate was collected by centrifugation and washed with methanol. The product obtained was dissolved in 5 ml of water, neutralised, and sodium chloride added to a concentration of 10% w/v and precipitated with 50 ml of methanol. The precipitate was collected by filtration, washed in methanol and dried in a vacuum oven at 35 °C. A total of 1.32 g product was obtained (yield 52.1%). The characteristics of the product obtained were as follows:
- Average molecular weight: 2903 Da.
- Anti-FXa activity: 193 IU/mg.
- Anti-FIIa activity: 9.3 IU/mg.

### EXAMPLE 2

50 g of sodium heparin were dissolved in 365 ml of water and 221 g of benzalkonium chloride solution in 50% w/v in water were added. The product was filtered, washed with water and lyophilised. 128.18 g benzalkonium heparinate were obtained.

5 g of benzalkonium heparinate obtained in the previous stage were dissolved in 15 ml dichloromethane and warmed to 35 °C. To this solution, BEMP was added in three portions:
- 0.86 ml of BEMP. The reaction was maintained at 35 °C for 8 hours.
- 0.86 ml of BEMP. The reaction was maintained at 35 °C for 16 hours.
- 0.86 ml of BEMP. The reaction was maintained at 35 °C for 8 hours.

To the solution, 0.50 ml hydrogen peroxide were added and the reaction maintained at 35 °C for 16 hours and then the product was precipitated with 30 ml of a solution of sodium acetate in methanol (10% w/v). The precipitate was collected by centrifugation and washed with methanol. The product obtained was dissolved in 2.5 ml of water, neutralised, and sodium chloride added to a concentration of 10% w/v and precipitated with 75 ml of methanol. The precipitate was collected by filtration, washed in methanol and dried in a vacuum oven at 35 °C. 1.40 g of product was obtained.

1.40 g of the depolymerised product was dissolved in 10 ml of water and 6.18 g of a solution of benzalkonium chloride at 50% w/v in water were added. The product was filtered, washed with water and lyophilised. 3.40 g of benzalkonium heparinate were obtained and were dissolved in 10 ml of dichloromethane and warmed to 35 °C. To this solution, BEMP was added in three portions:
- 0.58 ml of BEMP. The reaction was maintained at 35 °C for 8 hours.
- 0.58 ml of BEMP. The reaction was maintained at 35 °C for 16 hours.
- 0.58 ml of BEMP. The reaction was maintained at 35 °C for 8 hours.

To the solution, 0.34 ml hydrogen peroxide were added and the reaction maintained at 35 °C for 16 hours and then the product was precipitated with 20 ml of a solution of sodium acetate in methanol (10% w/v). The precipitate was collected by centrifugation and washed with methanol. The product obtained was dissolved in 1.7 ml of water, neutralised, and sodium chloride added to a concentration of 10% w/v and precipitated with 50 ml of methanol. The precipitate was collected by filtration, washed in methanol and dried in a vacuum oven at 35 °C. 0.85 g of product were obtained (yield 43.6%). The characteristics of the product obtained were as follows:
- Average molecular weight: 2,787 Da.
- Anti-FXa activity: 121.5 IU/mg.
- Anti-FIIa activity: 8.2 IU/mg.

### EXAMPLE 3

0.50 g of the product obtained in Example 2 were dissolved in 5 ml of water, neutralised, sodium chloride added to a concentration of 10% w/v and precipitated with 6 ml of methanol. The precipitate was collected by filtration, washed in methanol and dried in a vacuum oven at 35 °C. 0.13 g of product were obtained. The characteristics of the product obtained were as follows:
- Average molecular weight: 3,534 Da.
- Anti-FXa activity: 125.2 IU/mg.
- Anti-FIIa activity: 19.4 IU/mg.

The same steps of the previous paragraph were repeated, changing the base from BEMP to TTMG.

The characteristics of the product obtained were as follows:
- Average molecular weight: 3704 Da.
- Anti-FXa activity: 118.5 IU/mg.
- Anti-FIIa activity: 16.2 IU/mg.

### EXAMPLE 4. Comparative example

In this example, the effect of the change of base in the process of the invention was investigated. The same test was performed with Triton B and with BEMP to demonstrate the influence of the use of the base phosphazene in the process of the invention.
50 g of sodium heparin were dissolved in water and 221 g of benzalkonium chloride in water (50% w/v) were added. The product was filtered, washed with water and lyophilised. 128.18 g of benzalkonium heparinate were obtained.

25 g of benzalkonium heparinate obtained in the previous step were dissolved in 75 ml of dichloromethane and warmed to 35 °C. To this solution was added Triton B in three portions:
- 6.25 ml of Triton B. The reaction was maintained at 35 °C for 8 hours.
- 6.25 ml of Triton B. The reaction was maintained at 35 °C for 16 hours.
- 6.25 ml of Triton B. The reaction was maintained at 35 °C for 8 hours.

2.5 ml of hydrogen peroxide were added to the solution and then the product was purified to obtain the corresponding sodium salt. The product obtained was transalified to obtain the benzalkonium salt, in accordance with the conditions and proportions described above.

15.68 g of benzalkonium heparinate obtained in the previous step were dissolved in 47 ml of dichloromethane and warmed to 35 °C. To this solution was added Triton B in three portions:
- 3.92 ml of Triton B. The reaction was maintained at 35 °C for 8 hours.
- 3.92 ml of Triton B. The reaction was maintained at 35 °C for 16 hours.
- 3.92 ml of Triton B. The reaction was maintained at 35 °C for 8 hours.

The product obtained was characterised by:
- Average molecular weight: 2387 Da.
- Anti-FXa activity: 94 IU/mg.

The same test were performed but using the base BEMP for the depolymerisation.

5 g of benzalkonium heparinate were dissolved in 15 ml of dichloromethane and warmed to 35 °C. To this solution were added BEMP in three portions:
- 0.86 ml of BEMP. The reaction was maintained at 35 °C for 8 hours.
- 0.86 ml of BEMP. The reaction was maintained at 35 °C for 16 hours.
- 0.86 ml of BEMP. The reaction was maintained at 35 °C for 8 hours.

0.5 ml of hydrogen peroxide were added to the solution and then the product was purified to obtain the corresponding sodium salt. The product obtained was transalified to obtain the benzalkonium salt, in accordance with the conditions and proportions described above.

3.40 g of benzalkonium heparinate obtained in the previous step were dissolved in 10 ml of dichloromethane and warmed to 35 °C. To this solution were added BEMP in three portions:
- 0.58 ml of BEMP. The reaction was maintained at 35 °C for 8 hours.
- 0.58 ml of BEMP. The reaction was maintained at 35 °C for 16 hours.
- 0.58 ml of BEMP. The reaction was maintained at 35 °C for 8 hours.
The product obtained was characterised by:
- Average molecular weight: 2787 Da.
- Anti-FXa activity: 121.5 IU/mg.

This comparative example shows that the phosphazene base in the process significantly increase the anti-FXa activity of the product obtained.

The mean monosaccharide content in the samples was determined by Nuclear Magnetic Resonance (NMR), using quantitative two-dimensional 13C-1H HSQC assays, according to the method described by Marco Guerrini et al. The increase in resolution achieved with the second dimension enables quantification of the signals that overlap in the one-dimensional spectrum, this technique being of especial interest for studying complex carbohydrates. These molecules shows serious overlap problems in 1H one-dimensional spectra that make the determination of the areas of isolated peaks in the 1D difficult for quantification.

The amount of the D-glucuronic acid unit linked to N-sulfo-3-O-sulfo-D-glucosamine (G-(ANS,3S)) present in GAGs obtained from natural heparin can be directly related to their anti-Xa activity, as described by M. Guerrini et al. This disaccharide belongs to the pentasaccharide responsible for the interaction with antithrombin III and is only detected in the active sequences. The correlation signal of the anomeric carbon of this type of glucuronic acid with the hydrogen directly linked appears in a characteristic region and free of overlap in the HSQC spectrum and therefore can be used to quantify the proportion of the pentasaccharide in the sample.

The 13C-1H HSQC spectra of the product obtained by depolymerisation with Triton B and BEMP are shown in Figures 1 and 2 respectively. The 1H-13C correlation signal of the anomeric proton corresponding to the glucuronic acid unit of the pentasaccharide has been highlighted with a circle.

The measurement of the intensity of this signal in the spectra enable determining the proportion of pentasaccharide.

**Table 1. Different fractions with Triton B and BEMP.**

| FRACTIONS | EXAMPLE 4. DEPOLYMERISATION WITH TRITON B, (%) | EXAMPLE 4. DEPOLYMERISATION WITH BEMP (%) |
|---|---|---|
| ANS | 37.0 | 37.1 |
| ANAc | 2.5 | 1.8 |
| I2S | 28.9 | 28.7 |
| I | 1.4 | 2.3 |
| G | 4.7 | 5.7 |
| ANS, 3S | 1.4 | 2.7 |
| G-ANS 3S | 1.6 | 2.0 |

The results show that the use of the base BEMP rather than Triton B gives more selectivity in the depolymerisation reaction with higher preservation of the pentasaccharide activator of ATIII, so the % ANS,3S increases by almost 93% and the % G-ANS,3S increases by 25% in the product depolymerised with BEMP compared to that depolymerised with Triton B. This ensures that this pentasaccharide fraction is clearly preserved in the products obtained by the process of the invention, providing an improved activity in these products and ensuring the yield of the process of the invention as a result of the increase in selectivity of the reaction.

## Claims

1. Process for obtaining very low molecular weight heparin (VLMWH) and low molecular weight heparin (LMWH) **characterised in that** it comprises the steps of:
a) transalification of heparin of an alkali or alkaline earth metal into benzalkonium heparinate;
b) depolymerisation in organic medium with the addition of a phosphazene base or a base derived from guanidine; and
c) transalification to form a salt of an alkali or alkaline earth metal.

2. Process of claim 1 wherein between step b) and step c), the product obtained in step b) is treated with hydrogen peroxide.

3. Process of any of the claims 1 and 2 wherein the product obtained after step c) is purified.

4. Process of claim 3 wherein the product obtained after step c) is purified by dissolving in water at a concentration of between 7.5% and 12.5% (w/v), adjusting the pH to a value of between 6.5 and 7.25, addition of sodium chloride to a concentration of between 5% and 10% (w/v) and precipitation with methanol.

5. Process of claims 1-4 wherein the depolymerisation step is performed in dichloromethane at a temperature of between 20 and 40 °C.

6. Process of claim 1 wherein in step b), the weight:volume ratio between benzalkonium heparinate and the base for each addition is between 1:0.5 and 1:0.05.

7. Process of claim 1 wherein in step b), the weight:volume ration between benzalkonium heparinate and the base for each addition is between 1:0.3 and 1:0.1.

8. Process of claim 2 wherein in step c), the weight:volume ratio between benzalkonium heparinate and hydrogen peroxide is between 1:1 and 1:0.01.

9. Process of claim 2 wherein in step c), the weight:volume ratio between benzalkonium heparinate and hydrogen peroxide is between 1:0.2 and 1:0.05.

10. Process for obtaining very low molecular weight heparin (VLMWH) and low molecular weight heparin (LMWH) **characterised in that** consists the steps of:
a) transalification of heparin of an alkali or alkaline earth metal into benzalkonium heparinate;
b) depolymerisation in a solvent selected from: dichloromethane, dichloethane, chloroform, diemthylformamide and /or formamide at a temperature between 20°C and 40°C with the addition of a phosphazene base or a base derived from guanidine, in one or two successive additions wherein the weight: volume ratio between benzalkonium heparinate and the base for each addition is between 1:0.5 and 1:0.05; and
c) transalification to form a salt of an alkali or alkaline earth metal and wherein between step b) and step c), the product obtained in step b) is treated with inorganic peroxide.

11. Low molecular weight heparin (LMWH) or very low molecular weight heparin (VLMWH) obtained by the process of claim 10.

## Patentansprüche

1. Verfahren zur Gewinnung von Heparin mit sehr niedrigem Molekulargewicht (VLMWH) und Heparin mit niedrigem Molekulargewicht (LMWH), **dadurch gekennzeichnet, dass** es folgende Schritte umfasst:
a) Umsalzung von Heparin eines Alkali- oder Erdalkalimetalls zu Benzalkonium-Heparinat;
b) Depolymerisierung in einem organischen Medium unter Zugabe einer Phosphazen-Base oder einer von Guanidin abgeleiteten Base;
c) Umsalzung zur Bildung eines Salzes eines Alkali- oder Erdalkalimetalls.

2. Verfahren nach Anspruch 1, wobei das in Schritt b) erhaltene Produkt zwischen Schritt b) und Schritt c) mit Wasserstoffperoxid behandelt wird.

3. Verfahren nach einem der Ansprüche 1 und 2, wobei das nach Schritt c) erhaltene Produkt aufgereinigt wird.

4. Verfahren nach Anspruch 3, wobei das nach Schritt c) erhaltene Produkt durch Auflösen in Wasser in einer Konzentration von 7,5 % bis 12,5 % (w/v), Einstellen eines pH-Werts von 6,5 bis 7,25, Zugabe von Natriumchlorid in einer Konzentration von 5 % bis 10 % (w/v) und Ausfällen mit Methanol aufgereinigt wird.

5. Verfahren nach den Ansprüchen 1 bis 4, wobei der Depolymerisierungsschritt in Dichlormethan bei einer Temperatur von 20 bis 40 °C durchgeführt wird.

6. Verfahren nach Anspruch 1, wobei in Schritt b) das Verhältnis von Gewicht zu Volumen zwischen Benzalkonium-Heparinat und der Base bei jeder Zugabe zwischen 1:0,5 und 1:0,05 liegt.

7. Verfahren nach Anspruch 1, wobei in Schritt b) das Verhältnis von Gewicht zu Volumen zwischen Benzalkonium-Heparinat und der Base bei jeder Zugabe zwischen 1:0,3 und 1:0,1 liegt.

8. Verfahren nach Anspruch 2, wobei in Schritt c) das Verhältnis von Gewicht zu Volumen zwischen Benzalkonium-Heparinat und Wasserstoffperoxid zwischen 1:1 und 1:0,01 liegt.

9. Verfahren nach Anspruch 2, wobei in Schritt c) das Verhältnis von Gewicht zu Volumen zwischen Benzalkonium-Heparinat und Wasserstoffperoxid zwischen 1:0,2 und 1:0,05 liegt.

10. Verfahren zur Gewinnung von Heparin mit sehr niedrigem Molekulargewicht (VLMWH) und Heparin mit niedrigem Molekulargewicht (LMWH), **dadurch gekennzeichnet, dass** es aus folgenden Schritten besteht:
a) Umsalzung von Heparin eines Alkali- oder Erdalkalimetalls zu Benzalkonium-Heparinat;
b) Depolymerisierung in einem Lösungsmittel, das ausgewählt wird aus: Dichlormethan, Chloroform, Dimethylformamid und/oder Formamid, bei einer Temperatur von 20 °C bis 40 °C unter Zugabe einer Phosphazen-Base oder einer von Guanidin abgeleiteten Base in ein oder zwei Zugaben, wobei das Verhältnis von Gewicht zu Volumen zwischen Benzalkonium-Heparinat und der Base bei jeder Zugabe zwischen 1:0,5 und 1:0,05 liegt;
c) Umsalzung zur Bildung eines Salzes eines Alkali- oder Erdalkalimetalls und wobei das in Schritt b) erhaltene Produkt zwischen Schritt b) und Schritt c) mit anorganischem Peroxid behandelt wird.

11. Heparin mit niedrigem Molekulargewicht (LMWH) oder Heparin mit sehr niedrigem Molekulargewicht (VLMWH), das mit dem Verfahren nach Anspruch 10 erhalten wird.

## Revendications

1. Procédé d'obtention d'héparine de très bas poids moléculaire (HTBPM) et d'héparine de bas poids moléculaire (HBPM) **caractérisé en ce qu'**il comprend les étapes de :
a) trans-salification d'héparine d'un métal alcalin ou alcalino-terreux en héparinate de benzalkonium ;
b) dépolymérisation en milieu organique avec l'ajout d'une base de phosphazène ou d'une base dérivée de guanidine ; et
c) trans-salification pour former un sel d'un métal alcalin ou alcalino-terreux.

2. Procédé de la revendication 1, dans lequel entre l'étape b) et l'étape c), le produit obtenu dans l'étape b) est traité avec du peroxyde d'hydrogène.

3. Procédé de l'une quelconque des revendications 1 et 2, dans lequel le produit obtenu après l'étape c) est purifié.

4. Procédé de la revendication 3, dans lequel le produit obtenu après l'étape c) est purifié par dissolution dans de l'eau à une concentration comprise entre 7,5% et 12,5% (P/V), régulation du pH à une valeur comprise entre 6,5 et 7,25, ajout de chlorure de sodium à une concentration comprise entre 5% et 10% (P/V) et précipitation avec méthanol.

5. Procédé des revendications 1-4, dans lequel l'étape de dépolymérisation est réalisée dans du dichlorométhane à une température comprise entre 20 et 40°C.

6. Procédé de la revendication 1, dans lequel dans l'étape b), le rapport poids /volume entre héparinate de benzalkonium et la base pour chaque ajout est compris entre 1:0,5 et 1:0,05.

7. Procédé de la revendication 1, dans lequel dans l'étape b), le rapport poids /volume entre héparinate de benzalkonium et la base pour chaque ajout est compris entre 1:0,3 et 1:0,1.

8. Procédé de la revendication 2, dans lequel dans l'étape c), le rapport poids /volume entre héparinate de benzalkonium et peroxyde d'hydrogène est compris entre 1:1 et 1:0,01.

9. Procédé de la revendication 2, dans lequel dans l'étape c), le rapport poids /volume entre héparinate de benzalkonium et peroxyde d'hydrogène est compris entre 1:0,2 et 1:0,05.

10. Procédé d'obtention d'héparine de très bas poids moléculaire (HTBPM) et d'héparine de bas poids moléculaire (HBPM) **caractérisé en ce qu'**il comprend les étapes de :
a) trans-salification d'héparine d'un métal alcalin ou alcalino-terreux en héparinate de benzalkonium ;
b) dépolymérisation dans un solvant sélectionné parmi dichlorométhane, dichloroéthane, chloroforme, diméthylformamide et /ou formamide à une température comprise entre 20°C et 40°C avec l'ajout d'une base de phosphazène ou d'une base dérivée de guanidine, en un ou deux ajouts successifs où le rapport poids /volume entre héparinate de benzalkonium et la base pour chaque ajout est compris entre 1:0,5 et 1:0,05 ; et
c) trans-salification pour former un sel d'un métal alcalin ou alcalino-terreux et où entre l'étape b) et l'étape c), le produit obtenu dans l'étape b) est traité avec du peroxyde inorganique.

11. Héparine de bas poids moléculaire (HBPM) ou héparine de très bas poids moléculaire (HTBPM) obtenue par le procédé de la revendication 10.
